# EUROPEAN PATENT APPLICATION

(11) **EP 4 579 589 A1**
(43) Date of publication of application: **02.07.2025**
(21) Application number: 24753151.0
(22) Date of filing: 26.01.2024
(51) Int. Cl.: G06T 7/00, C12M 1/34, C12M 3/00, C12Q 1/04

(54) **APPARATUS FOR PRODUCING IDENTIFIER FOR USE IN CELL CLASSIFICATION, IDENTIFIER FOR USE IN CELL CLASSIFICATION, CELL CLASSIFICATION APPARATUS, AND CELL TREATMENT SYSTEM**

(30) Priority: 07.02.2023 JP 2023016496
(71) Applicant: Kataoka Corporation, Kyoto-shi, Kyoto 601-8203 (JP)
(72) Inventor: MATSUMOTO Junichi, Kyoto-shi, Kyoto 601-8203 (JP); KUSAKA Ayumi, Kyoto-shi, Kyoto 601-8203 (JP); MORISHITA Tadao, Kyoto-shi, Kyoto 601-8203 (JP)
(74) Representative: Seemann & Partner Patentanwälte mbB
(86) International application number: PCT/JP2024/002339
(87) International publication number: WO 2024/166707

(57) **Abstract**

The present disclosure provides an apparatus for generating an identifier that is for use in cell classification and is adapted to enable improved discrimination between target cells and non-target cells even in a region that is under the influence of a meniscus. An apparatus 100 for generating an identifier for use in cell classification according to the present disclosure includes: an acquisition section 611 that acquires a captured image of observed objects including cells in a culture vessel D; a classification data generation section 612 that generates classification data of the captured image on a pixel basis, the classification data including classifications of pixels of the captured image, the classifications including a region classification and an observed object classification, the region classification including at least two types of region classifications, and the observed object classification including at least three types of observed object classifications including a classification as being target cells and a classification as being non-target cells; and a generation section 613 that generates an identifier through machine learning using training data in which the captured image and the classification data are used as a set.

## Description

### TECHNICAL FIELD

The present disclosure relates to an apparatus for generating an identifier for use in cell classification, an identifier for use in cell classification, a cell classification apparatus, and a cell treatment system.

### BACKGROUND ART

In expansion culture of cells or differentiation induction from undifferentiated cells into differentiated cells performed using a culture tool, not only cells desired to be obtained (hereinafter also referred to as "target cells") but also undesired cells (hereinafter also referred to as "non-target cells") are included in the resulting cultured cells. Thus, in such expansion culture and differentiation induction, it has been common practice to sort the target cells by discriminating between the target cells and the non-target cells and removing the non-target cells.

The discrimination and the removal of the non-target cells are performed by a skilled operator (Patent Literature 1). However, the above-described discrimination and removal are laborious operations that have to be performed under a microscope, for example, and there is a problem in that the quality of the target cells to be obtained varies greatly depending on the skill level of the operator.

### Citation List

### Patent Literature

Patent Literature 1: Japanese Translation of PCT International Application Publication No. JP-T-2014-509192

### SUMMARY OF INVENTION

### Technical Problem

In light of the above circumstances, the inventors of the present disclosure studied a technique for generating an identifier using training data labeled with non-target cells and discriminating the non-target cells using the identifier. However, even with the use of such an identifier, there has been a problem in that target cells and non-target cells cannot be sufficiently discriminated from each other in some cases. In particular, in a region where a meniscus is formed in a culture tool, there has been a problem in that target cells and non-target cells cannot be sufficiently discriminated from each other owing to a bending phenomenon at the liquid surface of a liquid culture medium.

With the foregoing in mind, it is an object of the present disclosure to provide an identifier for use in cell classification, adapted to enable improved discrimination between target cells and non-target cells even in a region that is under the influence of a meniscus.

### Solution to Problem

In order to achieve the above object, the present disclosure provides an apparatus for generating an identifier for use in cell classification (hereinafter also referred to simply as "generation apparatus"), including:
the classification data including classifications of pixels of the captured image,
the classifications including a region classification and an observed object classification,
the region classification including at least two types of region classifications, and
the observed object classification including at least three types of observed object classifications including a classification as being target cells and a classification as being non-target cells; and
a generation section that generates an identifier through machine learning using training data in which the captured image and the classification data are used as a set.

The present disclosure also provides an identifier for use in cell classification (hereinafter also referred to simply as "identifier") generated through machine learning in order to assign, to each pixel of a captured image of observed objects including cells in a culture vessel, a classification of a region where the pixel is present in the culture vessel and a classification of an observed object that is present in the pixel, wherein
the identifier is generated through machine learning using training data in which the captured image and classification data are used as a set,
the classification data includes classifications of pixels of the captured image,
the classifications include a region classification and an observed object classification,
the region classification includes at least two types of region classifications, and
the observed object classification includes at least three types of observed object classifications, including a classification as being target cells and a classification as being non-target cells.

The present disclosure also provides a classification apparatus for cells (hereinafter also referred to simply as "classification apparatus"), including:
an identifier generated through machine learning in order to assign, to each pixel of a captured image of observed objects including cells in a culture vessel, a classification of a region where the pixel is present in the culture vessel and a classification of an observed object that is present in the pixel, the identifier being generated through machine learning using training data in which the captured image and classification data are used as a set,
   the classification data including classifications of pixels of the captured image,
   the classifications including a region classification and an observed object classification,
   the region classification including at least two types of region classifications, and
   the observed object classification including at least three types of observed object classifications including a classification as being target cells and a classification as being non-target cells;
an acquisition section that acquires the captured image of the observed objects including the cells in the culture vessel; and
a classification assigning section that assigns, to the pixels of the captured image acquired by the acquisition section, the classifications using the identifier.

The present disclosure also provides a cell treatment system including:
the cell classification apparatus according to the present disclosure; and
a laser irradiation device capable of irradiating a culture vessel with a laser,
wherein the laser irradiation device irradiates the culture vessel with a laser, thereby changing the state of at least one of cells to which a classification as being target cells has been assigned or cells to which a classification as being non-target cells has been assigned in the classification apparatus.

### Advantageous Effects of Invention

According to the present disclosure, improved discrimination between target cells and non-target cells can be achieved even in a region that is under the influence of a meniscus.

### BRIEF DESCRIPTION OF THE DRAWINGS

FIG. 1 is a schematic view showing an example of a generation apparatus (classification apparatus) of a first embodiment.
FIG. 2 is a block diagram showing an example of an arithmetic device in the generation apparatus (classification apparatus) of the first embodiment.
FIG. 3 is a flowchart illustrating a generation method of the first embodiment.
FIG. 4 shows: a photograph obtained by segmenting a culture vessel into a plurality of segments, capturing images of the respective segments, and integrating the thus-obtained segmented images using a camera of the first embodiment; magnified photographs showing a meniscus region and a non-meniscus region in the above photograph; and a schematic cross-sectional view showing an example of the state of the meniscus in the culture vessel.
FIG. 5 shows examples of phase-contrast microscope images acquires by an acquisition section in the first embodiment and schematic diagrams showing classification data including the region classification and the observed object classification.
FIG. 6 is a flowchart illustrating a classification method of the first embodiment.
FIG. 7 is a schematic diagram illustrating a procedure for assigning classifications to fluorescence microscope images, which are used as training data in combination with phase-contrast microscope images in a second embodiment.
FIG. 8 is a schematic view showing an example of the configuration of a cell treatment system of a third embodiment.
FIG. 9 is a flowchart illustrating a cell treatment method of the third embodiment.

### DESCRIPTION OF EMBODIMENTS

### <Definition>

As used herein, "cells" refers to cells or a construct containing cells. The cells may be, for example, cells, or a cell mass, tissue, or organ composed of cells. The cells may be, for example, cultured cells or cells isolated from a living body. Specific examples of the cells include: pluripotent stem cells such as iPS cells and ES cells; somatic stem cells such as hematopoietic stem cells and neural stem cells; progenitor cells; and somatic cells. The cells may be, for example, undifferentiated cells or differentiated cells (e.g., cells that have deviated from the undifferentiated state).

As used herein, "observation" refers to observation of an object to be observed. The "observation" may be, for example, observation with or without image capturing.

As used herein, "the state of cells" refers to whether the cells are alive or dead, the morphology of the cells, the gene expression in the cells, or the like. As used herein, a "change in the state of cells" refers to a change in whether the cells are alive or dead, the morphology of the cells, the gene expression in the cells, or the like. Specific examples of the "change in the state of cells" include: causing the cells to die; and inducing or suppressing the gene expression in the cells. The change in the gene expression can be caused, for example, using an optogenetic approach.

As used herein, an "optical axis direction" refers to a direction in which the optical axis (symmetry axis) of an imaging optical system extends and is also referred to as the "Z-axis direction". The optical axis direction can also be referred to as, for example, a direction orthogonal (perpendicular) to a surface on which an object to be observed is to be placed. Further, as used herein, the "X-axis direction" refers to one direction in a plane (XY plane) orthogonal to the optical axis direction, and the "Y-axis direction" refers to a direction that is orthogonal (perpendicular) to the X axis direction in the XY plane.

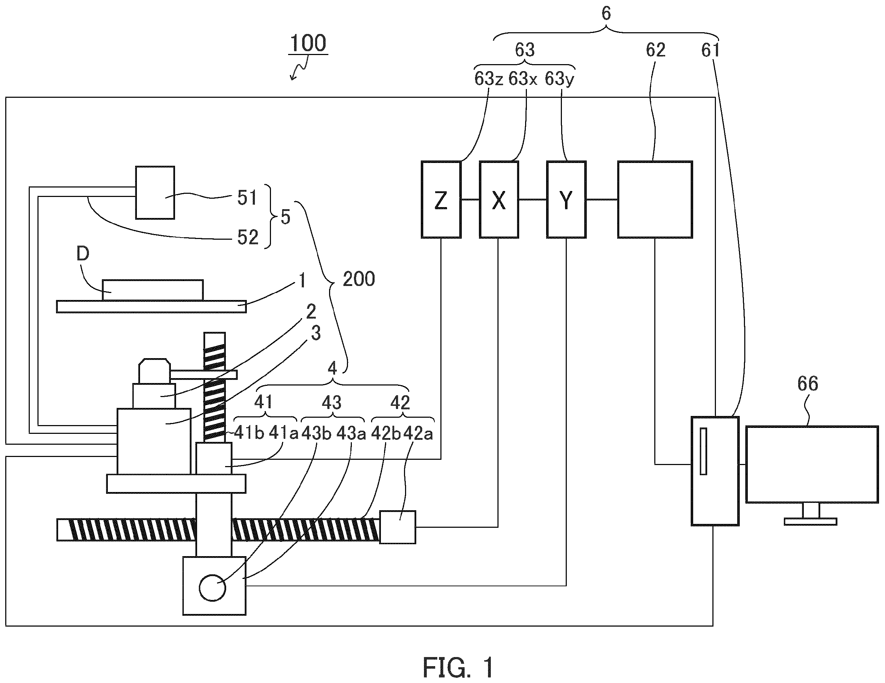

The present disclosure will be described in detail below with reference to the drawings. It is to be noted that the present disclosure is by no means limited by the following description. In FIGs. 1 to 9 to be described below, identical parts are given the same reference numerals, and duplicate explanations thereof may be omitted. In the drawings, the structure of each part may be shown in a simplified form as appropriate for the sake of convenience in explanation, and each part may be shown schematically with a dimensional ratio and the like that are different from the actual dimension ratio and the like. Descriptions regarding one embodiment can also be applied to another embodiment, and vice versa, unless otherwise stated.

### (First Embodiment)

The first embodiment relates to a generation apparatus, identifier, and classification apparatus according to the present disclosure.

The present embodiment describes an example of each of the generation apparatus, identifier, and classification apparatus. FIG. 1 is a schematic view of a generation apparatus 100 of the first embodiment. As shown in FIG. 1, the generation apparatus 100 includes an image capturing device 200 and a control unit 6 as its main components. The image capturing device 200 has a configuration similar to that of a phase-contrast microscope, and includes, as its main components, a stage 1 as a placement unit, an objective lens 2 as an imaging optical system, a camera 3 as an image capturing unit, a moving unit 4, and a lighting unit 5. The moving unit (driving section) 4 includes: a first moving unit, which is a Z-axis direction moving unit 41; and second moving units, which are an X-axis direction moving unit 42 and a Y-axis direction moving unit 43". The Z-axis direction moving unit 41 includes a Z-axis motor 41a and a Z-axis ball screw 41b. The X-axis direction moving unit 42 includes an X-axis motor 42a and an X-axis ball screw 42b. The Y-axis direction moving unit 43 includes a Y-axis motor 43a and a Y-axis ball screw 43b. The lighting unit 5 includes a light source 51 and a support member 52 for supporting the light source 51. The control unit 6 includes an arithmetic device 61, a sequencer (PLC) 62, and a motor driver 63. The configuration of the arithmetic device 61 will be described below. The motor driver 63 includes an X-axis direction motor driver 63x, a Y-axis direction motor driver 63y, and a Z-axis direction motor driver 63z. The generation apparatus 100 of the first embodiment also functions as a classification apparatus, as will be described below.

A culture vessel D containing cells, which is a component not included in the generation apparatus 100, is placed on the stage 1. The stage 1 may have any configuration as long as an object to be observed can be placed thereon. As a specific example, the placement unit may have a configuration of a placement unit in an optical observation device. The optical observation device may be, for example, a bright-field microscope, stereoscopic microscope, phase-contrast microscope, differential interference microscope, polarizing microscope, fluorescence microscope, confocal laser microscope, total internal reflection fluorescence microscope, or Raman microscope, and preferably is a phase-contrast microscope. A region for placing the culture vessel D in the stage 1 is adapted such that the culture vessel D can be observed via the objective lens 2 arranged below the stage 1. The region for placing the culture vessel D may be formed of a light-transmitting material such as glass, quartz, plastic, or resin, and a through hole may be formed in a part of the region.

The object to be observed is an object that is to be subjected to observation or image capturing using the image capturing device 200 of the generation apparatus 100. In the present embodiment, the object to be observed is the culture vessel D containing cells. However, the object to be observed may be any sample as long as the sample can be observed using an optical observation device. For example, the object to be observed may be a cell culture vessel, such as a dish, plate, or flask (cell culture flask), containing cells, or a glass slide with a sample set thereon.

The objective lens 2 forms an observed image of the culture vessel D as the object to be observed on the camera 3 as the image capturing unit. More specifically, the objective lens 2 forms an observed image of the cells in the culture vessel D on the camera 3. This allows the image capturing device 200 to perform observation and image capturing of the cells in the culture vessel D. Although the objective lens 2 constitutes the imaging optical system in the image capturing device 200, the imaging optical system is not limited as long as it can form an observed image of the object to be observed. The imaging optical system may employ, for example, a configuration of an imaging optical system in the above-described optical observation device. Although one objective lens 2 is used in the image capturing device 200, two or more objective lenses may be used. In this case, the magnifications of these objective lenses 2 may be the same or different from each other.

Although the objective lens 2 is arranged below the culture vessel D in the image capturing device 200, the objective lens 2 may be arranged above the culture vessel D. The position at which the objective lens 2 as the imaging optical system is arranged can be set as appropriate according to, for example, the type of the above-described optical observation device.

The camera 3 is configured to be capable of capturing an observed image of the culture vessel D as the object to be observed, and more specifically, capable of capturing an observed image of the cells in the culture vessel D. In the image capturing device 200, the camera 3 provided with an image sensor element is used as the image capturing unit. However, the image capturing unit may have any configuration as long as it can capture an observed image of the object to be observed. The image sensor element may be, for example, a known image sensor element, and specific examples thereof include elements such as a charge-coupled device (CCD) and a complementary metal oxide semiconductor (CMOS). Accordingly, the image capturing unit may be, for example, an image capturing device such as a camera provided with such an image sensor element.

The camera 3 is configured to capture an observed image of the culture vessel D as the object to be observed upon receipt of an image-capturing trigger signal transmitted from the arithmetic device 61 to be described below. The imaging time (exposure time) for single image capturing by the camera 3 can be set as appropriate according to, for example, the brightness of the object to be observed.

In the image capturing device 200, the camera 3 captures the observed image after the observed image has been formed on the camera 3, and the thus-obtained image (captured image) is displayed on a display device 66 provided outside the image capturing device 200. The image capturing device 200 may not only cause the observed image to be displayed on the display device 66 but also relay an image (primary image) obtained by the objective lens 2 to an ocular lens, through which the user of the image capturing device 200 observes the image. In this case, the image capturing device 200 includes, for example: a relay optical system that relays the primary image to the ocular lens; and the ocular lens. The relay optical system and the ocular lenses may have, for example, a configuration of a relay optical system and a configuration of an ocular lens of the above-described optical observation device, respectively. A specific example of the display device 66 will be described below.

The camera 3 as the image capturing unit transmits the captured image to the arithmetic device 61. In this case, it is preferable that the camera 3 transmits the captured image to the arithmetic device 61 after associating the position at which the image was captured (e.g., the coordinates such as the XY coordinates or the XYZ coordinates) with the captured image.

The moving unit 4 is capable of moving (driving) the objective lens 2 as the imaging optical system and the camera 3 as the image capturing unit. Although, in the image capturing device 200, the moving unit 4 is capable of moving the objective lens 2 and the camera 3, the moving unit 4 may be configured to be capable of moving only the objective lens 2. Alternatively, the moving unit 4 may be configured to be capable of moving, in addition to or instead of the objective lens 2, the stage 1 as the placement unit. In this case, the stage 1, which may be a motor-driven stage, a mechanical stage, or the like, may be integrated with the moving unit 4. When the moving unit 4 is configured to be capable of moving the objective lens 2 as the imaging optical system, it is possible to reduce, for example, shaking of the liquid surface of a liquid culture medium contained in the culture vessel D as the object to be observed, as compared with the case where the moving unit 4 is configured to be capable of moving the stage 1. With this configuration, the image capturing device 200 can suppress the occurrence of fluctuations of the intensity of illumination light, fluctuations of the focal point position, etc. caused by the shaking of the liquid surface, and this allows the camera 3 to obtain a plurality of images including an image in which the object to be observed is more precisely in focus (focused image).

The moving unit 4 includes the first moving unit (driving section) movable in the Z-axis direction, i.e., the optical axis direction, and the second moving units (driving sections) movable in the XY plane directions. In the moving unit 4, the first moving unit includes the Z-axis motor 41a and the Z-axis ball screw 41b. The second moving units includes the X-axis motor 42a and the X-axis ball screw 42b and the Y-axis motor 43a and the Y-axis ball screw 43b. As shown in FIG. 1, the Z-axis ball screw 41b, the X-axis ball screw 42b, and the Y-axis ball screw 43b are mounted so as to extend in the Z-, X-, and Y-axis directions, respectively. The Z-axis ball screw 41b is connected to a nut on the X-axis ball screw 42b so as to be movable on the X-axis ball screw 42b in the X-axis direction. The X-axis ball screw 42b is connected to a nut on the Y-axis ball screw 43b so as to be movable on the Y-axis ball screw 43b in the Y-axis direction. The objective lens 2 and the camera 3 are connected to a nut on the Z-axis ball screw 41b so as to be movable on the Z-axis ball screw 41b in the Z-axis (optical axis) direction. The Z-axis motor 41a, the X-axis motor 42a, and the Y-axis motor 43a are connected to the Z-axis direction motor driver 63z, the X-axis direction motor driver 63x, and the Y-axis direction motor driver 63y, respectively. Accordingly, in the image capturing device 200, driving signals to be described below are transmitted by the motor driver 63 to be described below. Then, the Z-axis motor 41a, the X-axis motor 42a, and the Y-axis motor 43a are driven based on the driving signals, whereby the objective lens 2 and the camera 3 are moved to the XYZ coordinates specified by the driving signals. The Z-axis motor 41a, the X-axis motor 42a, and the Y-axis motor 43a may be, for example, position-controllable motors, and specific examples thereof include stepping motors.

In the image capturing device 200, the moving unit 4 need only be capable of moving at least one of the placement unit and the imaging optical system, and may have a configuration of a corresponding component in the above-described optical observation device. Although the moving unit 4 is constituted by the ball screws and the motors, linear motors may be used to constitute the moving unit 4, and further, carriages may be used in combination. When the objective lens 2 includes a focus adjustment mechanism such as a lens extending mechanism, the focus adjustment mechanism may be used as the first moving unit included in the moving unit 4.

The moving unit 4 need only be configured to be capable of moving at least one of the placement unit and the imaging optical system in the optical axis (Z-axis) direction, and may be configured to be capable of moving at least one of the placement unit and the imaging optical system further in at least one of the X-axis direction and the Y-axis direction. The movement in the X-axis direction and the Y-axis directions also can be referred to as, for example, movement in a plane (XY plane) that is perpendicular (orthogonal) to the optical axis direction.

Next, the lighting unit 5 illuminates the culture vessel D, the object to be observed, placed on the stage 1. The lighting unit 5 includes the light source 51 capable of illuminating the object to be observed. In addition to the light source 51, the lighting unit 5 may further include an illumination optical system configured to guide illumination light emitted from the light source 51 to the object to be observed. The light source 51 may be, for example, a halogen lamp, tungsten lamp, or light emitting diode (LED). The illumination optical system may have, for example, a configuration of an illumination optical system in the above-described optical observation device.

In the generation apparatus 100, the lighting unit 5 is arranged so as to face the objective lens 2 and the camera 3 via the stage 1. In other words, the lighting unit 5 is arranged above the stage 1. However, the position at which the lighting unit 5 is arranged is not limited thereto, and can be set as appropriate according to the type of the above-described optical observation device. For example, the lighting unit 5 may be arranged below the stage 1. The lighting unit 5 is coupled to the camera 3 via the support member 52, and thus moves in conjunction with the movement of the camera 3. It is to be noted that the present disclosure is not limited thereto. The lighting unit 5 may be configured to be movable independently of the camera 3, or may be configured not to be movable. When the lighting unit 5 is configured to be movable independently of the camera 3, the lighting unit 5 preferably moves so as to be coaxially aligned with the camera 3 in the Z-axis direction. When the lighting unit 5 moves in conjunction with the camera 3 in the generation apparatus 100, an image capturing region of the culture vessel D that is to be observed and whose image is to be captured by the objective lens 2 and the camera 3 can be favorably illuminated. The image capturing region refers to a region subjected to image capturing by the image capturing device 200.

As described above, the control unit 6 includes the arithmetic device 61, PLC 62, and motor driver 63. In the generation apparatus 100, the arithmetic device 61 in the control unit 6 works together with the PLC 62 and the motor driver 63 to function as respective parts including a movement control unit, an image capturing control unit, an acquisition section 611, a classification data generation section 612, and a generation section 613. It is to be noted that the present disclosure is not limited thereto, and the arithmetic device 61 alone may function as the movement control unit, the image capturing control unit, the acquisition section 611, the classification data generation section 612, and the generation section 613, or alternatively, the arithmetic device 61 and the motor driver 63 may work together to function as the movement control unit, the image capturing control unit, the acquisition section 611, the classification data generation section 612, and the generation section 613.

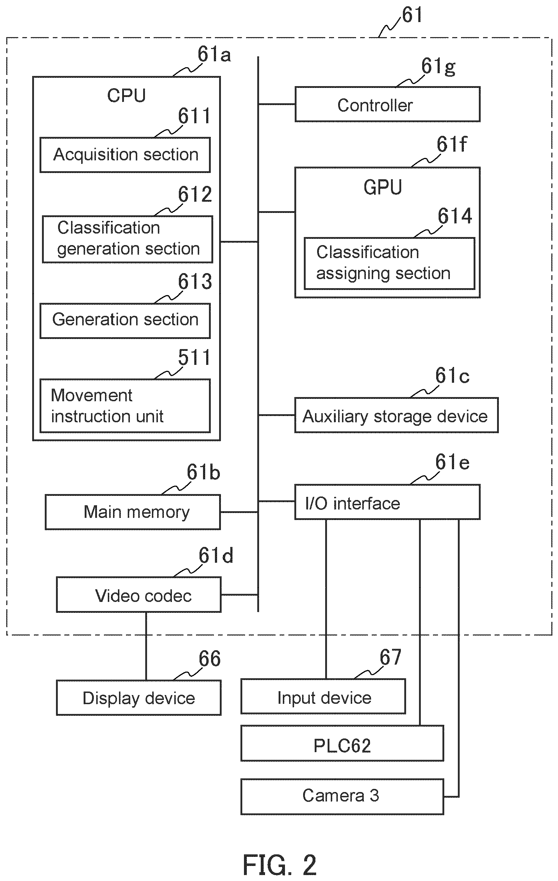

The arithmetic device 61 has a configuration similar to that of a personal computer, server computer, workstation, or the like. FIG. 2 is a block diagram showing an example of the arithmetic device 61 in the image capturing device 200. As shown in FIG. 2, the arithmetic device 61 includes a central processing unit (CPU) 61a, a main memory (main storage device) 61b, an auxiliary storage device 61c, a video codec 61d, an input-output (I/O) interface 61e, a GPU 61f, and other components, and they operate in cooperation with each other under the control by a controller (system controller, I/O controller, or the like) 61g. Examples of the auxiliary storage device 61c include storage means such as a flash memory and a hard disk drive. Although the arithmetic device 61 includes the CPU 61a and the GPU 61f as arithmetic means (processors), the arithmetic device 61 may include either one of them. The video codec 61d includes: a graphics processing unit (GPU) that generates an image to be displayed on a screen based on a drawing instruction received from the CPU 61a and transmits the screen signals to, for example, the display device 66 or the like provided outside the image capturing device 200; and a video memory or the like for temporarily storing data concerning the screen and the image. The I/O interface 61e is a device that is communicably connected to the PLC 62 and the camera 3 in order to control them or to obtain information such as images. The I/O interface 61e may include a servo driver (servo controller). The I/O interface 61e may be connected to, for example, an input device 67 provided outside the image capturing device 200. The display device 66 may be, for example, a monitor that outputs data in the form of a video (e.g., various image display devices such as a liquid crystal display (LCD) and a cathode ray tube (CRT) display). The input device 67 may be, for example, a pointing device such as a touch panel, track-pad, or mouse, a keyboard, or a depressible button, each operable by the user with his/her fingers. The GPU 61f processes images (captured images) input from the camera 3 of the image capture device 200.

A program to be executed by the arithmetic device 61 and each piece of information have been stored in the auxiliary storage device 61c. At the time of executing the program, the program is read into the main memory 61b and decoded by the CPU 61a. Then, according to the program, the arithmetic device 61 functions as the respective parts including the movement control unit, the image capturing control unit, the acquisition section 611, the classification data generation section 612, and the generation section 613. Thus, in the generation apparatus 100, the CPU 61a functions as the movement control unit, the image capturing control unit, the acquisition section 611, the classification data generation section 612, and the generation section 613. The functions of the respective parts will be described below.

An identifier to be used by the arithmetic device 61 is stored in the auxiliary memory device 61c. Accordingly, when the generation apparatus 100 functions as the classification apparatus, i.e., when the arithmetic device 61 assigns classifications, the identifier is read into the main memory 61b at the time of executing the identifier and decoded by the GPU 61f. Thus, the GPU 61f functions as a classification assigning section 614. The functions of the respective parts will be described below.

As shown in FIG. 1, the PLC 62 is connected to the motor driver 63, more specifically, to the Z-axis direction motor driver 63z, the X-axis direction motor driver 63x, and Y-axis direction motor driver 63y of the motor driver 63. The PLC 62 may be, for example, a programmable logic controller or a sequencer. The PLC 62 converts information specifying the position to which the objective lens 2 is to be moved (hereinafter referred to as "destination position") transmitted by the arithmetic device 61 into motor commands for operating the motor driver 63 that controls the Z-axis motor 41a, the X-axis motor 42a, and the Y-axis motor 43a. Subsequently, the PLC 62 drives the Z-axis motor 41a, the X-axis motor 42a, and the Y-axis motor 43a via the motor driver 63, and when the objective lens 2 has reached the specified destination position, the PLC 62 then transmits information that the movement of the objective lens 2 has been completed to the arithmetic device 61.

In the image capturing device 200, the arithmetic device 61 controls the motor driver 63 via the PLC 62. That is, in the arithmetic device 61, the PLC 62 and the motor driver 63 work together to function as the movement control unit for controlling the movement caused by the moving unit 4. It is to be noted that the present disclosure is not limited thereto, and the arithmetic device 61 may directly control the motor driver 63. In this case, the arithmetic device 61 includes, for example: a motor controller; a microcontroller having a motor control function; and a field-programmable gate array (FPGA).

As shown in FIG. 1, the motor driver 63 includes the Z-axis direction motor driver 63z, the X-axis direction motor driver 63x, and the Y-axis direction motor driver 63y. The Z-axis direction motor driver 63z, the X-axis direction motor driver 63x, and the Y-axis direction motor driver 63y are connected to the Z-axis motor 41a, the X-axis motor 42a, and the Y-axis motor 43a, respectively. The Z-axis direction motor driver 63z, the X-axis direction motor driver 63x, and the Y-axis direction motor driver 63y drive the Z-axis motor 41a, the X-axis motor 42a, and the Y-axis motor 43a, respectively, by transmitting driving signals thereto based on the motor commands transmitted from the PLC 62. The driving signals may be, for example, two-phase pulse signals.

In the generation apparatus 100, the arithmetic device 61 directly controls image capturing performed by the camera 3. More specifically, in the generation apparatus 100, the arithmetic device 61 transmits an image-capturing trigger signal for commanding the camera 3 to capture an image.

Next, a generation method of the first embodiment performed using the generation apparatus 100 of the first embodiment will be described.

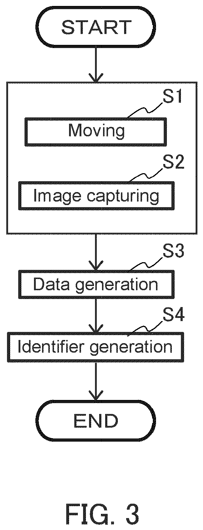

FIG. 3 is a flowchart illustrating the generation method of the first embodiment. As shown in FIG. 3, the generation method of the first embodiment includes Step S1 (moving), Step S2 (image capturing), Step S3 (data generation), and Step S4 (identifier generation). In the generation method of the first embodiment, Steps S1 and S2 are performed concurrently.

In Step S1, the objective lens 2, which forms an observed image of the culture vessel D as the object to be observed, is moved from a movement start position to a movement end position along the optical axis direction of the objective lens 2. Specifically, in Step S1, the movement control unit of the arithmetic device 61 transmits information (e.g., XYZ coordinates) specifying the destination position of the objective lens 2 to the PLC 62. Then, based on the information specifying the destination position of the objective lens 2, the PLC 62 and the motor driver 63 work together to control the movement of the objective lens 2 by the moving unit 4. As a result, in Step S1, the objective lens 2 is moved by the moving unit 4 from the movement start position to the movement end position along the Z-axis direction.

The movement start position refers to a position at which the movement in the optical axis direction is started, and more specifically refers to a position at which the movement in the optical axis direction is started and at which the image capturing control unit controls the start of image capturing of the observed image. The movement end position refers to a position at which the movement in the optical axis direction is ended, and more specifically refers to a position at which the movement in the optical axis direction is ended and at which an image capturing instruction unit 512 to be described below controls the end of image capturing of the observed image. The movement start position and the movement end position may be set, for example, by the user of the image capturing device 200 in advance or based on the coordinates of the inner bottom surface of the culture vessel D or the coordinates of the stage 1. The movement start position and the movement end position are represented by, for example, XYZ coordinates. The distance between the movement start position and the movement end position in the optical axis (Z-axis) direction can be set, for example, based on the thickness of the object to be observed, i.e., the length of the object to be observed in the optical axis direction. In the case where the object to be observed is cells, the distance between the movement start position and the movement end position in the optical axis direction is, for example, from 0.2 to 0.5 mm. The movement start position and the movement end position can be set, for example, so as to include a position in the Z-axis direction at which a focused image is obtained in a region that is not under the influence of a meniscus.

In the generation method of the first embodiment, the objective lens 2 as the imaging optical system and the camera 3 are moved in Step S1. It is to be noted that the present disclosure is not limited thereto. In Step S1, the stage 1 as the placement unit may be moved, or both the stage 1 and the objective lens 2 may be moved.

Next, in Step S2, the camera 3 captures a plurality of observed images during the movement in the optical axis direction. Specifically, in Step S2, the image capturing control unit of the arithmetic device 61 transmits an ON signal to the PLC 62 and the Z-axis direction motor driver 63z. Based on the ON signal, the PLC 62 and the Z-axis direction motor driver 63z work together to cause the camera 3 to capture a plurality of observed images of the cells in the culture vessel D during the movement of the objective lens 2 in the optical axis direction in Step S1. Then, in Step S2, the camera 3 transmits the plurality of captured images to the arithmetic device 61 after associating the XYZ coordinates of the objective lens 2 at the time when the image was captured with each captured image. The thus-transmitted captured images are stored in the auxiliary storage device 61c of the arithmetic device 61. Although the plurality of captured images are stored in Step S2, one captured image selected by the user or the like may be stored.

The image capturing control unit controls image capturing performed by the camera 3 such that the camera 3 captures a plurality of observed images during the movement in the optical axis direction. The number of observed images to be captured can be determined according to the moving distance in the optical axis direction and the distance between the movement start position and the movement end position. The number of observed images to be captured need only be two or more, and the upper limit thereof is not limited to any particular value. The image capturing instruction unit 512 preferably controls the camera 3 so as to capture a plurality of observed images by capturing an observed image using the above-described image sensor element every time the camera 3 has moved a predetermined distance during the movement in the optical axis direction. With this configuration, the image capturing device 200 can select a focused image in which the object to be observed is more precisely in focus in selection performed by the selection unit 514 to be described below. Although a plurality of images are captured and a focused image is selected therefrom in Step S2, the present disclosure is not limited thereto, and only one image may be captured. In this case, in Step S2, it is preferable that, for example, the user adjusts the position of the objective lens 2 such that the object to be observed in the culture vessel D comes into focus. By performing image capturing in this state, the image capturing device 200 can capture a focused image in which the object to be observed is more precisely in focus.

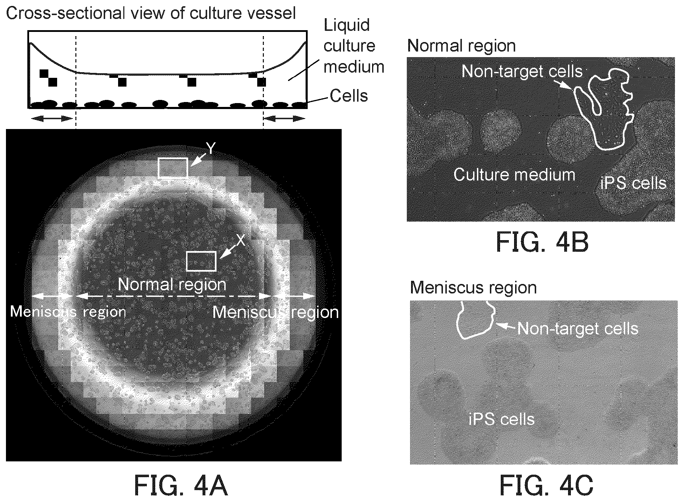

Next, in Steps S3 and S4, to pixels of the captured images stored in the auxiliary storage device 51c, classifications of the pixels are assigned, and an identifier is generated through machine learning using training data in which the captured image and the classification data are used as a set. Steps S3 and S4, which are characteristic processes of the first embodiment, will be described with reference to FIG. 4. FIG. 4 shows a photograph (phase-contrast microscope image) obtained by segmenting the culture vessel D in which iPS cells were subjected to expansion culture into a plurality of segments, capturing images of the respective segments, and integrating the thus-obtained segmented images using the camera 3; magnified photographs showing a region where a meniscus is formed (meniscus region) and a region where a meniscus is not formed (normal region, non-meniscus region) in the above-described photograph, and a schematic cross-sectional view showing an example of the state of the meniscus in the culture vessel D. In FIG. 4A, the upper row is a schematic cross-sectional view of the culture vessel D, and the lower row is the phase-contrast microscope image of the entire culture vessel D. FIG. 4B is a magnified image of a normal region indicated by the arrow X in FIG. 4A, and FIG. 4C is a magnified image of a meniscus region indicated by the arrow Y in FIG. 4A. With reference to FIG. 4, an example where the target cells are iPS cells and the non-target cells are cells other than iPS cells will be described below.

As can be seen in FIGs. 4A to 4C, the normal region exhibits a high contrast in the phase-contrast microscope image, and the observed image of the target cells (iPS cells) and the observed image of the non-target cells (cells other than the iPS cells) can be identified clearly. On the other hand, in the meniscus region, the phase contrast deteriorates in the phase-contrast microscope image, thereby causing a change in the observed image of the target cells (iPS cells) and the observed image of the non-target cells (cells other than iPS cells). In particular, when comparing the observed images between the normal region and the meniscus region, a marked change is observed between the observed images of the non-target cells. On this account, it was presumed that an identifier generated using training data labeled with the non-target cells would cause a problem in that it cannot sufficiently distinguish the non-target cells in the culture tool, especially the non-target cells in the meniscus region.

The inventors of the present disclosure have found out that, as can be seen in FIGs. 4B and 4C, in each of the normal region and the meniscus region, the change in observed image is relatively small between the observed images of the target cells and between the observed image of the non-target cells. On the basis of this finding, the inventors of the present invention devised a method of, when assigning classifications to each pixel of a captured image, assigning a classification of an observed object, such as cells, present in the pixel in combination with a region classification of the pixel. This allows the identifier to be parameterized in consideration of the information concerning the region. Therefore, the generation apparatus 100 of the first embodiment can achieve improved discrimination between target cells and non-target cells even in a region that is under the influence of a meniscus.

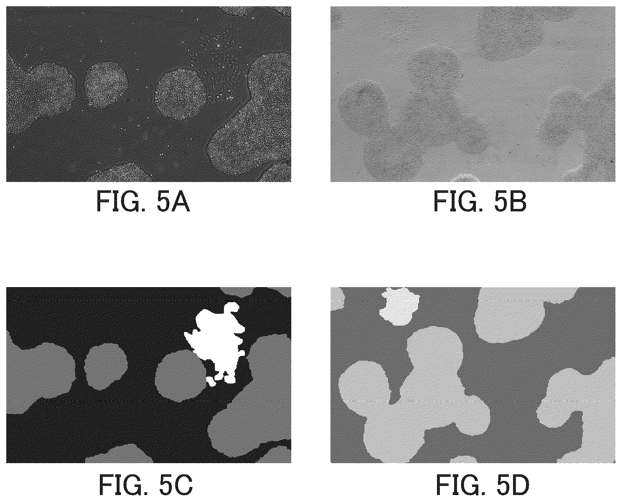

Next, Step S3 will be described in detail with reference to FIG. 5. FIG. 5 shows examples of phase-contrast microscope images acquires by the acquisition section 611 and schematic diagrams showing classification data including the region classifications and the observed object classifications. FIG. 5A is a phase-contrast microscope image of the normal region, FIG. 5B is a phase-contrast microscope image of the meniscus region, FIG. 5C shows classification data schematically showing classifications in the normal region, and FIG. 5D shows classification data schematically showing classifications in the meniscus region. Although FIGs. 5C and 5D use images to schematically represent the classification data, the classification data is data in which the classifications are associated with the coordinates in the images.

In Step S3, the phase-contrast microscope images of FIGs. 5A and 5B are used to create classification data to which the classifications shown in FIGs. 5C and 5D are assigned. In Step S3, first, the acquisition section 611 acquires a phase-contrast microscope image that is an observed image stored in the auxiliary storage device 61c. Next, in Step S3, the classification data generation section 612 assigns classifications to each pixel of the acquired phase-contrast microscope image. In Step S3, the classifications can be assigned by, for example, associating information input by the user. Specifically, in Step S3, first, the phase-contrast microscope image to which the classifications are to be assigned is displayed on the display device 66. Then, the user assigns the region classification and the observed object classification to each pixel of the phase-contrast microscope image using the input device 67. In the generation apparatus 100 of the first embodiment, the user assigns, as the region classification, a classification as being the meniscus region or a classification as being the normal region (non-meniscus region). The user also assigns, as the observed object classification, a classification as being the target cells (iPS cells), a classification as being the non-target cells (cells other than iPS cells), or a classification as being another observed object (e.g., as being a culture medium). In the generation apparatus 100 of the first embodiment, based on the information input via the input device 67, the classification data generation section 612 associates the coordinates of the pixel (e.g., XY coordinates) with the region classification and the observed object classification, thereby generating classification data. In this manner, in Step S3, classification data to which the classifications shown in FIGs. 5C and 5D are assigned can be generated from the phase-contrast microscope images of FIGs. 5A and 5B.

Next, in the case where the object to be observed is segmented into a plurality of regions (segmented regions) that can be captured in a single field of view of the camera 3 and images of the respective segmented regions of the object to be observed are captured, in Step S1, a movement instruction unit 511 of the arithmetic device 61 transmits, to the PLC 62, information (e.g., XYZ coordinates) specifying the destination position of the objective lens 2 for image capturing of a subsequent segmented region. Then, the PLC 62 and the motor driver 63 work together to control the movement of the objective lens 2 by the moving unit 4 based on the information specifying the destination position of the objective lens 2, and the objective lens 2 moves to a newly specified segmented region. The movement to the newly specified segmented region is preferably movement on the XY plane and more preferably movement on the XY plane to a newly specified adjacent segmented region. After the movement to the newly specified segmented region, the generation apparatus 100 of the first embodiment performs Steps S1 to S3 in the same manner as described above. Then, the generation apparatus 100 of the first embodiment repeats the movement to a newly specified segmented region and the processes of Steps S1 to S3 in the newly specified segmented region until a segmented region whose image has not yet been captured no longer remains.

Next, in Step S4, an identifier is generated using the phase-contrast microscope image and the classification data created in Step S3. Specifically, in Step S4, training data is generated by the generation section 613 using the phase-contrast microscope image and the classification data as a pair. Then, the generation section 613 generates an identifier through machine learning using the training data. For the machine learning, a semantic segmentation model such as U-Net can be used, for example. The number of sets of training data is not limited to any particular value, and can be set by the user according to, for example, the discrimination accuracy of the resulting identifier. In this manner, Step S4 can generate an identifier capable of assigning, to each pixel of the captured image of the observed object including cells in the culture vessel D, a classification of a region where the pixel is present in the culture vessel D and a classification of an observed object that is present in the pixel. The thus-obtained identifier is then stored in the auxiliary storage device 61c, whereby Step S4 is completed.

Next, a classification method of the first embodiment performed using the generation apparatus 100 of the first embodiment will be described.

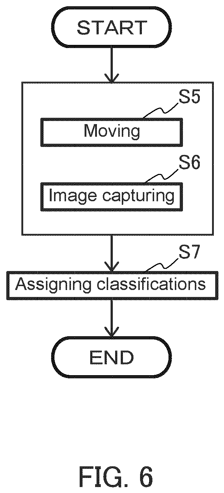

FIG. 6 is a flowchart illustrating the classification method of the first embodiment. As shown in FIG. 6, the classification method of the first embodiment includes Step S5 (moving), Step S6 (image capturing), and Step S7 (assigning classifications). In the classification method of the first embodiment, Steps S5 and S6 are performed concurrently. Although Step S7 is performed after Steps S5 and S6 in the classification method of the first embodiment, the present disclosure is not limited thereto, and Steps S5, S6, and S7 may be performed concurrently.

First, in the classification method of the first embodiment, Steps S5 and S6 are performed in the same manner as Steps S1 and S2, respectively.

Next, in Step S7, using the identifier obtained in Step S4, the above-described classifications are assigned to the pixels of the captured image stored in the auxiliary storage device 51c. In Step S7, first, the acquisition section 611 acquires a phase-contrast microscope image, which is the observed image stored in the auxiliary storage device 61c. Then, in Step S7, the classification assigning section 614 assigns, to each pixel of the acquired phase-contrast microscope image, the above-described classifications concerning the region and the type of the observed object using the identifier. Specifically, in Step S7, the captured image and the identifier are input to the classification assigning section 614, and the classifications concerning the region and the type of the observed object are assigned to each pixel of the captured image. In this manner, in Step S7, data in which the coordinates (e.g., XY coordinates) of the pixels are associated with the classification as being the target cells and the classification as being the non-target cells can be generated, whereby information for discriminating between the target cells and the non-target cells in the captured image can be acquires. The data concerning the classifications assigned to each pixel of the captured image is then stored in the auxiliary storage device 61c, whereby Step S7 is completed.

After images of all the segmented regions have been captured, the generation apparatus 100 of the first embodiment may generate a focused image of the entire culture vessel D from a plurality of captured images of the respective segmented regions. The integration can be achieved by arranging the respective captured images according to the XYZ coordinates associated with the captured images of the respective segmented regions. In this manner, the generation apparatus 100 of the first embodiment can obtain a captured image of the entire culture vessel D. In this case, the generation apparatus 100 of the first embodiment may perform Steps S3 and S4 with respect to the captured image of the entire culture vessel D. Also, the generation apparatus 100 of the first embodiment may perform Step S7 with respect to the captured image of the entire culture vessel D.

Although a phase-contrast microscope is used as the image capturing device 200 in the generation apparatus 100 of the first embodiment, the present disclosure is not limited thereto, and any of the above-described other optical observation devices may be used or the phase-contrast microscope may be used in combination with any other optical observation device. Examples of the above-described combination include the combination of the phase-contrast microscope and the fluorescence microscope.

### (Effects of First Embodiment)

In the generation apparatus 100 of the first embodiment, when assigning classifications to each pixel of the captured image, the classification data generation section 612 assigns a classification of an observed object, such as cells, present in the pixel in combination with a region classification of the pixel. Then, in the generation apparatus 100 of the first embodiment, the generation section 613 generates an identifier using training data in which the captured image and the classification data including the region classification and the observed object classification are used as a set. Accordingly, the generation apparatus 100 of the first embodiment can generate an identifier that is parameterized in consideration of the information on the region. Therefore, when assigning classifications to a captured image, the identifier obtained by the generation apparatus 100 of the first embodiment can assign an observed object classification in consideration of the region in the culture vessel D, thereby enabling improved discrimination between the target cells and the non-target cells.

The generation apparatus 100 of the first embodiment has been described above with reference to an example where, in the observed object classification, the target cells are iPS cells and the non-target cells are cells other than the iPS cells. It is to be noted that the present disclosure is not limited thereto, and the combination of the target cells and the non-target cells can be set freely. Examples of the combination of the target cells and the non-target cells (arbitrary order) include the combination of cells that are differentiated from undifferentiated cells (progenitor cells) in a differentiation induction system and the undifferentiated cells.

In the generation apparatus 100 of the first embodiment, the observed object classification includes one type of classification for the target cells and for the non-target cells. It is to be noted that the present disclosure is not limited thereto, and there may be two or more types of classifications for the target cells and for the non-target cells. When there are two or more types of target cells and non-target cells, the generation apparatus 100 of the first embodiment can generate an identifier having an improved discrimination accuracy by setting classifications for the respective types of target cells and non-target cells and assigning, to the different types of target cells and non-target cells, classifications as being the corresponding target cells and non-target cells by the classification data generation section 612. Examples of the types of target cells and non-target cells include the type of cells, the type of morphology that the cells exhibit (e.g., aggregation), the size of the cells, the size of a colony, and the expression of a specific protein.

Although the image capturing device 200 is included in the generation apparatus 100 of first embodiment, the image capturing device 200 is an optional component and need not necessarily be included. In this case, in the generation apparatus 100, for example, the acquisition section 611 acquires captured images stored outside the generation apparatus 100 by the image capturing device 200, and the classification data generation section 612 and the generation section 613 generate an identifier using the captured images acquired by the acquisition section 611.

The generation apparatus 100 of the first embodiment may further include a treatment apparatus for treating cells to which the classification as being the target cells or the non-target cells has been assigned. With this configuration, the generation apparatus 100 of the first embodiment can change the state of at least one of the target cells and the non-target cells to a desired state. The treatment may be, for example, a treatment using light such as a laser, a heat treatment, or a treatment using a chemical substance.

Although the generation apparatus 100 of the first embodiment uses all the captured images captured by the image capturing device 200, the present disclosure is not limited thereto, and the generation apparatus 100 may generate the identifier using some of the captured images or any desired number of captured images. When some of the captured images are used, the generation apparatus 100 can achieve improved discrimination between the target cells and the non-target cells by removing, for example, captured images that are not well captured and captured images that include dirt or the like and thus are not suitable for use as training data before learning. Also, when some of the captured images are used, for example, the generation apparatus 100 can adjust the ratio of the target cells and the non-target cells in the captured images and thus can prevent an imbalance in the number of pixels input as the training data between the target cells and the non-target cell. Although the generation apparatus 100 of the first embodiment generates the identifier using the captured images derived from the single culture vessel D, the present disclosure is not limited thereto, and the generation apparatus 100 may generate an identifier using captured images derived from a plurality of culture vessels D, i.e., different culture vessels D.

Although the generation apparatus 100 of the present embodiment also serves as a classification apparatus, the present disclosure is not limited thereto, and the generation apparatus 100 and the classification apparatus may be separate apparatuses.

### (Second Embodiment)

In the generation apparatus 100 of the first embodiment, when assigning classifications to a captured image, the classifications input by the user are associated with the captured image to assign the classifications. In light of the foregoing, a generation apparatus 100A of the second embodiment is a generation apparatus configured such that target cells and non-target cells that have been fluorescently stained in advance are used and a classification data generation section 612 uses fluorescence microscope images including the target cells and the non-target cells to assign a classification as being the target cells and a classification as being the non-target cells.

The generation apparatus 100A of the second embodiment corresponds to the generation apparatus 100 of the first embodiment, adapted such that the image capturing device 200 further includes the configuration of a fluorescence microscope in addition to the configuration of the phase-contrast microscope. The fluorescence microscope is, for example, a microscope to which a known fluorescence observation method is applied, and may be, for example, an epi-illumination fluorescence microscope or a transmission fluorescence microscope. In the present embodiment, an epi-illumination fluorescence microscope is used as the fluorescence microscope. The epi-illumination fluorescence microscope is a fluorescence microscope configured to irradiate a sample with excitation light by epi-illumination. More specifically, according to the epi-illumination fluorescence microscope, a sample is irradiated with light having an excitation wavelength using a dichroic mirror, and then, fluorescence derived from the sample and the excitation light scattered by the sample are guided to an eyepiece or an image sensor element. Further, fluorescence desired to be obtained is extracted using an absorption filter that transmits only light having the wavelength of the desired fluorescence, and the thus-extracted fluorescence is observed. In Step S2, the generation apparatus 100A captures phase-contrast microscope images by the phase-contrast microscope, and thereafter, captures fluorescence microscope images by the fluorescence microscope. Specifically, the generation apparatus 100A captures phase-contrast microscope images of respective segmented regions in the same manner as the generation apparatus 100 of the first embodiment. Next, the generation apparatus 100A acquires fluorescence microscope images of the respective segmented regions in the same manner as that for acquiring the phase-contrast microscope images, except that, instead of the phase-contrast microscope image, the fluorescence microscope is used as the image capturing device 200. It is preferable to capture one fluorescence microscope image for each segmented region in order to suppress, for example, fading of fluorescence. Except for the above, the generation apparatus 100A of the second embodiment has the same configuration as the generation apparatus 100 of the first embodiment, and the description on the configuration of the generation apparatus 100 also applies to the generation apparatus 100A. The phase-contrast microscope images and the fluorescence microscope images may be captured concurrently. In this case, the generation apparatus 100A captures a phase-contrast microscope image after the movement to a newly specified segmented region. Subsequently, the generation apparatus 100A captures a fluorescence microscope image for the same segmented region. The generation apparatus 100A repeats the movement to a newly specified segmented region and image capturing in the same manner until a segmented region whose image has not yet been captured no longer remains.

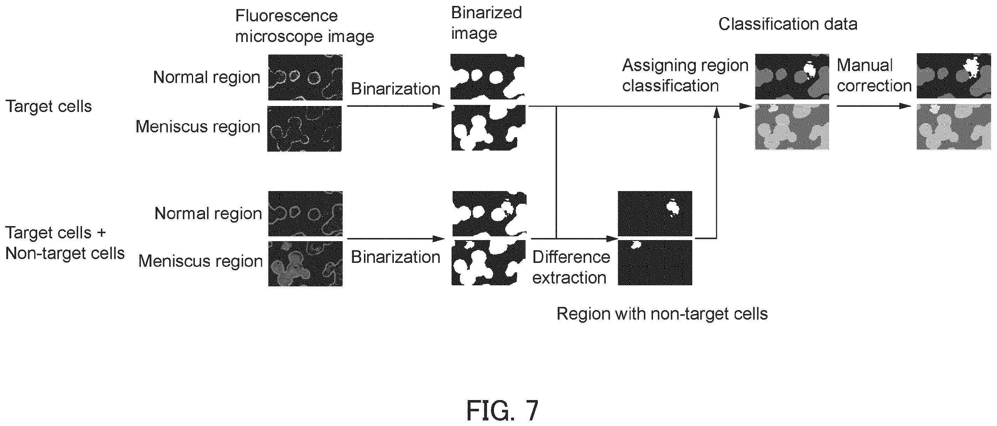

Steps S3 and S4 will be described more specifically with reference to FIG. 7. FIG. 7 is a schematic diagram illustrating a procedure for assigning the above-described classifications to fluorescence microscope images, which are used as training data in combination with phase-contrast microscope images. As shown in FIG. 7, cells in a culture vessel D are stained in advance with a fluorescent staining reagent that stains only iPS cells as target cells, and with a fluorescent staining reagent that stains iPS cells as the target cells and cells other than iPS cells as non-target cells. In Step S3, fluorescence microscope images obtained by capturing the cells in the culture vessel D that have been fluorescently stained in advance using the fluorescence microscope of the image capturing device 200 are used.

First, in Step S3, a fluorescence microscope image obtained by capturing the fluorescently stained target cells is binarized to extract regions where the target cells are present. The binarization may be performed, for example, based on designation by the user via an input device 67, using the Otsu's method, or using artificial intelligence (AI). To the thus-extracted regions where the target cells are present, a classification as being the target cells is assigned. When the binarization is performed using AI, part of the process of Step S3, i.e., the binarization, is performed using a GPU 61f.

Next, in Step S3, using the fluorescence microscope image obtained by capturing the fluorescently stained target cells and a fluorescence microscope image obtained by capturing the fluorescently stained target cells and the fluorescently stained non-target cells (i.e., all the cells), regions where the non-target cells are present are extracted. Specifically, the fluorescence microscope image of all the cells is binarized to extract the regions where all the cells are present. Subsequently, by extracting the difference between the regions where all the cells are present and the regions where the target cells are present, the regions where the non-target cells are present are extracted. Further, to the thus-extracted regions where the non-target cells are present, a classification as being the non-target cells is assigned. Then, to regions where neither the classification as being the target cells nor the classification as being the non-target cells has been assigned, a classification as being a culture medium (or a background) is assigned.

Further, a region classification is also assigned concurrently with the classification as being the target cells and the classification as being the non-target cells. The region where a meniscus is formed depends on the shape of the culture vessel D and the amount of the culture medium. Thus, the region where a meniscus is formed is stored in an arithmetic device 61 in advance. Then, in Step S3, with reference to previously set information concerning the region where a meniscus is formed, a classification as being the meniscus region is assigned to the region where a meniscus is formed in the above-described fluorescence microscope images, and a classification as being a normal region is assigned to a region where a meniscus is not formed. In this manner, the classification data can be generated in Step S3. In Step S3, for example, for the above-described classification data, the user may correct at least one of the classifications of the pixels and the observed object classification via the input device 67.

Then, in Step S4, a generation section 613 generates an identifier through machine learning using training data in which the phase-contrast microscope images and the classification data generated from fluorescence microscope images corresponding to the phase-contrast microscope images are used as a set. The thus-obtained identifier is then stored in an auxiliary storage device 61c, whereby Step S4 is completed.

In the generation apparatus 100A of the second embodiment, the classification data generation section 612 can specify the regions where the target cells are present and the regions where the non-target cells are present using the fluorescence microscope images, and can assign the observed object classification to the fluorescence microscope images. Thus, the generation apparatus 100A of the second embodiment can assign classifications to captured images used for the training data without requiring the user to input the classifications.

### (Third Embodiment)

In the generation apparatus 100 of the first embodiment and the generation apparatus 100A of the second embodiment, an identifier for assigning classifications of cells is generated using phase-contrast microscope images or using phase-contrast microscope images and fluorescence microscope images, and classifications are assigned to cells in the phase-contrast microscope images. A cell processing system 300 of the third embodiment will be described with reference to an example where, of the above-described cells, the non-target cells are treated by the cell processing system 300 utilizing the classifications assigned by the identifier.

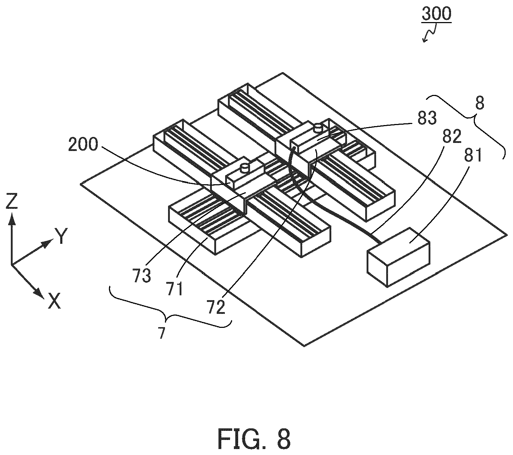

The cell treatment system 300 of the third embodiment includes a generation apparatus 100, a moving unit (driving section) 7, and a laser irradiation unit (laser emitter) 8. As shown in FIG. 8, the moving unit 7 has an XY stage 71 and carriages 72 and 73. The XY stage 71 includes a common Y-axis direction rail and two X-axis direction rails, and the two X-axis direction rails are movably arranged on the common Y-axis direction rail. The carriages 72 and 73 are movably arranged on the two X-axis direction rails, respectively. The laser irradiation unit 8 includes a laser source 81, an optical fiber 82, and a laser emission section 83. The optical fiber 82 optically connects the laser source 81 and the laser emission section 83. An image capturing device 200 of the generation apparatus 100 is arranged on the carriage 73 of the moving unit 7. The laser emission section 83 of the laser irradiation unit 8 is arranged on the carriage 72. With this configuration, by moving the carriages 72 and 73 on the XY stage 71, the image capturing device 200 and the laser irradiation unit 8 can perform image capturing and laser irradiation, respectively, with respect to any position in a culture vessel D. In the cell treatment system 300, a control unit 6 of the generation apparatus 100 is connected to the laser irradiation unit 8, and controls laser irradiation by the laser irradiation unit 8.

Next, a cell treatment method of the third embodiment performed using the cell treatment system 300 of the third embodiment will be described.

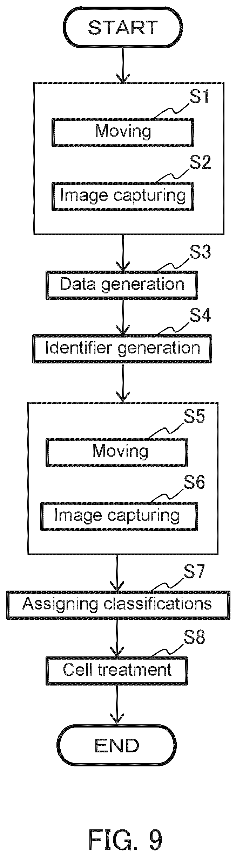

FIG. 9 is a flowchart illustrating a cell treatment method of the third embodiment. As shown in FIG. 9, the cell treatment method of the third embodiment further includes Step S8 (cell treatment) in addition to the respective steps in the generation method and the classification method of the first embodiment.

First, Steps S1 to S4 are performed in the same manner as in the generation method of the first embodiment using a culture vessel D1 containing cells for use in the generation of an identifier to generate an identifier capable of assigning, to each pixel of the captured image of the observed object including cells in the culture vessel D1, a classification of a region where the pixel is present in the culture vessel D1 and a classification of an observed object that is present in the pixel.

Next, using a culture vessel D2 containing cells to be treated, Steps S5 to S7 are performed in the same manner as in the classification method of the first embodiment. In this manner, the classifications are assigned to each pixel of captured images of the culture vessel D2 using the identifier. Although different culture vessels are used as the culture vessels D1 and D2, the same culture vessel may be used as the culture vessels D1 and D2.

Next, in Step S8, based on data concerning the classifications assigned in Step S7, non-target cells are irradiated with a laser using the laser irradiation unit 8 to kill the non-target cells. In this manner, in Step S8, the non-target cells in the culture vessel D2 are removed, thereby allowing only the target cells to remain. Specifically, in Step S8, the classifications obtained in Step S7 are read from an auxiliary memory device 61c and superimposed on the captured images obtained in Step S6. The captured images with the classifications superimposed thereon are then output to the display device 66. The user may correct the classifications based on the captured images with the classifications superimposed thereon. Next, the user inputs an instruction to start laser irradiation by the laser irradiation unit 8 via an input device 67. Then, the control unit 6 drives the XY stage 71 and the carriage 72 of the moving unit 7 to move the laser irradiation unit 8 to a position where the cells can be irradiated with a laser emitted from the laser emission section 83. Subsequently, the control unit 6 turns on the laser irradiation by the laser irradiation unit 8 to cause the non-target cells in the culture vessel D to be irradiated with a laser emitted from the laser emission section 83, whereby the non-target cells are killed. The control unit 6 repeatedly performs the same treatment for positions corresponding to pixels to which the classification as being the non-target cells has been assigned. The non-target cells in the culture vessel D2 are then removed, whereby Step S8 is completed. In Step S8, the classification as being the non-target cells may be input by the user, and a treatment using a laser may be performed with respect to the non-target cells specified by the input from the user.

In the second embodiment, an example where the image capturing device 200 of the generation apparatus 100 of the first embodiment includes the configuration of a fluorescence microscope in addition to the configuration of a phase-contrast microscope has been described. It is to be noted that the present disclosure is not limited thereto, and the image capturing device 200 may be a microscope having a phase-contrast microscope function and a fluorescence microscope function and the phase-contrast microscope function and the fluorescence microscope function may be switched in used.

While the present invention has been described above with reference to exemplary embodiments, the present invention is by no means limited to the above embodiments. Various changes and modifications that may become apparent to those skilled in the art may be made in the configuration and specifics of the present disclosure without departing from the scope of the present disclosure.

This application is based upon and claims the benefit of priority from Japanese Patent Application No. 2023-016496 filed on February 7, 2023, the entire disclosure of which is incorporated herein by reference.

### <Supplementary Notes>

The whole or part of the exemplary embodiments and examples disclosed above can be described as, but not limited to, the following Supplementary Notes.

### <Apparatus for Generating Identifier for Use in Cell Classification> (Supplementary Note 1)

An apparatus for generating an identifier for use in cell classification, including:
an acquisition section that acquires a captured image of observed objects including cells in a culture vessel;
a classification data generation section that generates classification data of the captured image on a pixel basis,
   the classification data including a region classification and an observed object classification,
   the region classification including at least two types of region classifications, and
   the observed object classification including at least three types of observed object classifications including a classification as being target cells and a classification as being non-target cells; and
a generation section that generates an identifier through machine learning using training data in which the captured image and the classification data are used as a set.

### (Supplementary Note 2)

The generation apparatus according to Supplementary Note 1, wherein
the acquisition section acquires a phase-contrast microscope image and a fluorescence microscope image of the cells in the culture vessel as captured images,
in the fluorescence microscope image, the cells are fluorescently labeled so as to be distinguishable,
the classification data generation section uses the fluorescence microscope image as a captured image and generates the classification data of the fluorescence microscope image on a pixel basis, and
the generation section generates the identifier through machine learning using training data in which the phase-contrast microscope image and the classification data are used as a set.

### (Supplementary Note 3)

The generation apparatus according to Supplementary Note 2, wherein
the fluorescence microscope image includes:
a first fluorescence microscope image in which both the target cells and the non-target cells are labeled with a fluorescent label so as to be distinguishable from other observed objects; and
a second fluorescence microscope image in which the target cells are labeled with a fluorescent label so as to be distinguishable from other observed objects; and
the classification data generation section generates the classification data by:
   comparing the first fluorescence microscope image with the second fluorescence microscope image to extract a pixel in which the non-target cells are present and classifying the extracted pixel as being the non-target cells in terms of the observed object classification,
   extracting, from the second fluorescence microscope image, a pixel in which the target cells are present and classifying the extracted pixel as being the target cells in terms of the observed object classification, and
   extracting a region where neither the target cells nor the non-target cells are present as a pixel in which other observed objects are present and classifying the extracted pixel as being an observed object that does not fall within the classification as being the target cells or the classification as being the non-target cells in terms of the observed object classification.

### (Supplementary Note 4)

The generation apparatus according to Supplementary Note 2 or 3, wherein
the acquisition section includes:
a phase-contrast microscope that acquires a phase-contrast microscope image of the culture vessel; and
a fluorescence microscope that acquires a fluorescence microscope image of the culture vessel.

### (Supplementary Note 5)

The generation apparatus according to Supplementary Note 1, wherein
the captured image is a phase-contrast microscope image.

### (Supplementary Note 6)

The generation apparatus according to Supplementary Note 5, wherein
the acquisition section includes a phase-contrast microscope that acquires a phase-contrast microscope image of the culture vessel.

### (Supplementary Note 7)

The generation apparatus according to any one of Supplementary Notes 1 to 6, wherein
the observed object classification further includes a classification as being a culture medium, and
the classification data generation section uses the classification as being the culture medium as an observed object classification other than the classification as being the target cells and the classification as being the non-target cells to generate the classification data.

### (Supplementary Note 8)

The generation apparatus according to any one of Supplementary Notes 1 to 7, wherein
the acquisition section acquires segmented images that are obtained by segmenting the culture vessel into a plurality of segments and capturing images of the respective segments.

### (Supplementary Note 9)

The generation apparatus according to any one of Supplementary Notes 1 to 8, wherein
the region classification includes a classification as being a meniscus region and a classification as being a non-meniscus region.

### (Supplementary Note 10)

The generation apparatus according to any one of Supplementary Notes 1 to 9, wherein
the target cells are undifferentiated cells or differentiated cells.

### (Supplementary Note 11)

The generation apparatus according to Supplementary Note 10, wherein
the undifferentiated cells are iPS cells or progenitor cells.

### <Identifier for Use in Cell Classification>

### (Supplementary Note 12)

An identifier for use in cell classification, the identifier being generated through machine learning in order to assign, to each pixel of a captured image of observed objects including cells in a culture vessel, a classification of a region where the pixel is present in the culture vessel and a classification of an observed object that is present in the pixel, wherein
the identifier is generated through machine learning using training data in which the captured image and classification data are used as a set,
the classification data includes classifications of pixels of the captured image,
the classifications include a region classification and an observed object classification,
the region classification includes at least two types of region classifications, and
the observed object classification includes at least three types of observed object classifications, including a classification as being target cells and a classification as being non-target cells.

### (Supplementary Note 13)

The identifier according to Supplementary Note 12, wherein
the captured image includes a phase-contrast microscope image and a fluorescence microscope image of cells in the culture vessel, and
the identifier is generated through machine learning using training data in which the phase-contrast microscope image and, as the classification data, classification data generated from the fluorescence microscope image are used as a set.

### (Supplementary Note 14)

The identifier according to Supplementary Note 12, wherein
the captured image is a phase-contrast microscope image.

### (Supplementary Note 15)

The identifier according to any one of Supplementary Notes 12 to 14, wherein
the observed object classification further includes a classification as being a culture medium.

### (Supplementary Note 16)

The identifier according to any one of Supplementary Notes 12 to 15, wherein
the region classification includes a classification as being a meniscus region and a classification as being a non-meniscus region.

### (Supplementary Note 17)

The identifier according to any one of Supplementary Notes 12 or 16, wherein
the target cells are undifferentiated cells or differentiated cells.

### (Supplementary Note 18)

The identifier according to Supplementary Note 17, wherein
the undifferentiated cells are iPS cells or progenitor cells.

### <Classification Apparatus for Cells>

### (Supplementary Note 19)

A classification apparatus for cells, including:
an identifier generated through machine learning in order to assign, to each pixel of a captured image of observed objects including cells in a culture vessel, a classification of a region where the pixel is present in the culture vessel and a classification of an observed object that is present in the pixel, the identifier being generated through machine learning using training data in which the captured image and classification data are used as a set,
   the classification data including classifications of pixels of the captured image,
   the classifications including a region classification and an observed object classification,
   the region classification including at least two types of region classifications, and
   the observed object classification including at least three types of observed object classifications including a classification as being target cells and a classification as being non-target cells;
an acquisition section that acquires the captured image of the observed objects including the cells in the culture vessel; and
a classification assigning section that assigns, to the pixels of the captured image acquired by the acquisition section, the classifications using the identifier.

### (Supplementary Note 20)

The classification apparatus according to Supplementary Note 19, wherein
the acquisition section includes a phase-contrast microscope that acquires a phase-contrast microscope image of the culture vessel.

### (Supplementary Note 21)

The classification apparatus according to Supplementary Note 19 or 20, wherein
the captured image used in the training data includes a phase-contrast microscope image and a fluorescence microscope image of cells in the culture vessel, and
the identifier is generated through machine learning using training data in which the phase-contrast microscope image and, as the classification data, classification data generated from the fluorescence microscope image are used as a set.

### (Supplementary Note 22)

The classification apparatus according to Supplementary Note 19 or 20, wherein
the captured image used as the training data is a phase-contrast microscope image.

### (Supplementary Note 23)

The classification apparatus according to any one of Supplementary Notes 19 to 22, wherein
the observed object classification further includes a classification as being a culture medium.

### (Supplementary Note 24)

The classification apparatus according to any one of Supplementary Notes 19 to 23, wherein
the region classification includes a classification as being a meniscus region and a classification as being a non-meniscus region.

### (Supplementary Note 25)

The classification apparatus according to any one of Supplementary Notes 19 or 24, wherein
the target cells are undifferentiated cells or differentiated cells.

### (Supplementary Note 25)

The classification apparatus according to Supplementary Note 25, wherein
the undifferentiated cells are iPS cells or progenitor cells.

### <Cell Treatment System>

### (Supplementary Note 27)

A cell treatment system including:
the cell classification apparatus according to any one of Supplementary Notes 19 to 26; and
a laser irradiation device capable of irradiating a culture vessel with a laser,
wherein the laser irradiation device irradiates the culture vessel with a laser, thereby changing the state of at least one of cells to which a classification as being target cells has been assigned or cells to which a classification as being non-target cells has been assigned in the classification apparatus.

### Reference Signs List

- 1:: Stage
- 2:: Objective lens
- 3:: Camera
- 4:: Moving unit
- 41:: X-axis direction moving unit
- 41a:: X-axis motor
- 41b:: X-axis ball screw
- 42:: Y-axis direction moving unit
- 42a:: Y-axis motor
- 42b:: Y-axis ball screw
- 43:: Z-axis direction moving unit
- 43a:: Z-axis motor
- 43b:: Z-axis ball screw
- 5:: Lighting unit
- 51:: Light source
- 52:: Support member
- 6:: Control unit
- 61:: Arithmetic device
- 611:: Acquisition section
- 612:: Classification data generation section
- 613:: Generation section
- 614:: Classification assigning section
- 61a:: CPU
- 61b:: Main memory
- 61c:: Auxiliary storage device
- 61d:: Video codec
- 61e:: I/O interface
- 61f:: GPU
- 61g:: Controller
- 62:: PLC
- 63:: Motor driver
- 63x:: X-axis direction motor driver
- 63y:: Y-axis direction motor driver
- 63z:: Z-axis direction motor driver
- 66:: Display device
- 67:: Input device
- 7:: Moving unit
- 71:: XY stage
- 72, 73:: Carriage
- 8:: Laser irradiation unit
- 81:: Laser source
- 82:: Optical fiber
- 83:: Laser emission section
- 100, 100A:: Generation apparatus
- 200:: Image capturing device
- 300:: Cell treatment system

## Claims

1. An apparatus for generating an identifier for use in cell classification, comprising:
an acquisition section that acquires a captured image of observed objects including cells in a culture vessel;
a classification data generation section that generates classification data of the captured image on a pixel basis,
the classification data including classifications of pixels of the captured image,
the classifications including a region classification and an observed object classification,
the region classification including at least two types of region classifications, and
the observed object classification including at least three types of observed object classifications including a classification as being target cells and a classification as being non-target cells; and
a generation section that generates an identifier through machine learning using training data in which the captured image and the classification data are used as a set.

2. The generation apparatus according to claim 1, wherein
the acquisition section acquires a phase-contrast microscope image and a fluorescence microscope image of the cells in the culture vessel as captured images,
in the fluorescence microscope image, the cells are fluorescently labeled so as to be distinguishable,
the classification data generation section uses the fluorescence microscope image as a captured image and generates the classification data of the fluorescence microscope image on a pixel basis, and
the generation section generates the identifier through machine learning using training data in which the phase-contrast microscope image and the classification data are used as a set.

3. The generation apparatus according to claim 2, wherein
the fluorescence microscope image includes:
a first fluorescence microscope image in which both the target cells and the non-target cells are labeled with a fluorescent label so as to be distinguishable from other observed objects; and
a second fluorescence microscope image in which the target cells are labeled with a fluorescent label so as to be distinguishable from other observed objects; and
the classification data generation section generates the classification data by:
comparing the first fluorescence microscope image with the second fluorescence microscope image to extract a pixel in which the non-target cells are present and classifying the extracted pixel as being the non-target cells in terms of the observed object classification,
extracting, from the second fluorescence microscope image, a pixel in which the target cells are present and classifying the extracted pixel as being the target cells in terms of the observed object classification, and
extracting a region where neither the target cells nor the non-target cells are present as a pixel in which other observed objects are present and classifying the extracted pixel as being an observed object that does not fall within the classification as being the target cells or the classification as being the non-target cells in terms of the observed object classification.

4. The generation apparatus according to claim 2 or 3, wherein
the acquisition section includes:
a phase-contrast microscope that acquires a phase-contrast microscope image of the culture vessel; and
a fluorescence microscope that acquires a fluorescence microscope image of the culture vessel.

5. The generation apparatus according to claim 1, wherein
the captured image is a phase-contrast microscope image.

6. The generation apparatus according to claim 5, wherein
the acquisition section includes a phase-contrast microscope that acquires a phase-contrast microscope image of the culture vessel.

7. The generation apparatus according to claim 1 or 2, wherein
the observed object classification further includes a classification as being a culture medium, and
the classification data generation section uses the classification as being the culture medium as an observed object classification other than the classification as being the target cells and the classification as being the non-target cells to generate the classification data.

8. The generation apparatus according to claim 1 or 2, wherein
the acquisition section acquires segmented images that are obtained by segmenting the culture vessel into a plurality of segments and capturing images of the respective segments.

9. The generation apparatus according to claim 1 or 2, wherein
the region classification includes a classification as being a meniscus region and a classification as being a non-meniscus region.

10. The generation apparatus according to claim 1 or 2, wherein
the target cells are undifferentiated cells or differentiated cells.

11. The generation apparatus according to claim 10, wherein
the undifferentiated cells are iPS cells or progenitor cells.

12. An identifier for use in cell classification, the identifier being generated through machine learning in order to assign, to each pixel of a captured image of observed objects including cells in a culture vessel, a classification of a region where the pixel is present in the culture vessel and a classification of an observed object that is present in the pixel, wherein
the identifier is generated through machine learning using training data in which the captured image and classification data are used as a set,
the classification data includes classifications of pixels of the captured image,
the classifications include a region classification and an observed object classification,
the region classification includes at least two types of region classifications, and
the observed object classification includes at least three types of observed object classifications, including a classification as being target cells and a classification as being non-target cells.

13. The identifier according to claim 12, wherein
the captured image includes a phase-contrast microscope image and a fluorescence microscope image of cells in the culture vessel, and
the identifier is generated through machine learning using training data in which the phase-contrast microscope image and, as the classification data, classification data generated from the fluorescence microscope image are used as a set.

14. The identifier according to claim 12, wherein
the captured image is a phase-contrast microscope image.

15. The identifier according to claim 12 or 13, wherein
the observed object classification further includes a classification as being a culture medium.

16. The identifier according to claim 12 or 13, wherein
the region classification includes a classification as being a meniscus region and a classification as being a non-meniscus region.

17. The identifier according to claim 12 or 13, wherein
the target cells are undifferentiated cells or differentiated cells.

18. The identifier according to claim 17, wherein
the undifferentiated cells are iPS cells or progenitor cells.

19. A classification apparatus for cells, comprising:
an identifier generated through machine learning in order to assign, to each pixel of a captured image of observed objects including cells in a culture vessel, a classification of a region where the pixel is present in the culture vessel and a classification of an observed object that is present in the pixel, the identifier being generated through machine learning using training data in which the captured image and classification data are used as a set,
the classification data including classifications of pixels of the captured image,
the classifications including a region classification and an observed object classification,
the region classification including at least two types of region classifications, and
the observed object classification including at least three types of observed object classifications including a classification as being target cells and a classification as being non-target cells;
an acquisition section that acquires the captured image of the observed objects including the cells in the culture vessel; and
a classification assigning section that assigns, to the pixels of the captured image acquired by the acquisition section, the classifications using the identifier.

20. The classification apparatus according to claim 19, wherein
the acquisition section includes a phase-contrast microscope that acquires a phase-contrast microscope image of the culture vessel.

21. The classification apparatus according to claim 19 or 20, wherein
the captured image used in the training data includes a phase-contrast microscope image and a fluorescence microscope image of cells in the culture vessel, and
the identifier is generated through machine learning using training data in which the phase-contrast microscope image and, as the classification data, classification data generated from the fluorescence microscope image are used as a set.

22. The classification apparatus according to claim 19 or 20, wherein
the captured image used as the training data is a phase-contrast microscope image.

23. The classification apparatus according to claim 19 or 20, wherein
the observed object classification further includes a classification as being a culture medium.

24. The classification apparatus according to claim 19 or 20, wherein
the region classification includes a classification as being a meniscus region and a classification as being a non-meniscus region.

25. The classification apparatus according to claim 19 or 20, wherein
the target cells are undifferentiated cells or differentiated cells.

26. The classification apparatus according to claim 25, wherein
the undifferentiated cells are iPS cells or progenitor cells.

27. A cell treatment system comprising:
the cell classification apparatus according to claim 19 or 20; and
a laser irradiation device capable of irradiating a culture vessel with a laser,
wherein the laser irradiation device irradiates the culture vessel with a laser, thereby changing the state of at least one of cells to which a classification as being target cells has been assigned or cells to which a classification as being non-target cells has been assigned in the classification apparatus.
